# FASCICULE DE BREVET EUROPEEN

(11) **EP 2 976 122 B1**
(45) Date de publication et mention de la délivrance du brevet: **03.05.2017**
(21) Numéro de dépôt: 14720645.2
(22) Date de dépôt: 18.03.2014
(51) Int. Cl.: A61M 15/00

(54) **DISPOSITIF DE DISTRIBUTION DE PRODUIT FLUIDE**
VORRICHTUNG ZUR VERTEILUNG EINES FLÜSSIGPRODUKTS
FLUID PRODUCTS DISPENSING DEVICE

(30) Priorité: 19.03.2013 FR 1352423
(43) Date de publication de la demande: 27.01.2016
(73) Titulaire: Aptar France SAS, 27110 Le Neubourg (FR)
(72) Inventeur: COLOMB, Arnaud, 78480 Verneuil Sur Seine (FR); KIRNIAK, Maxime, 76230 Bois-Guillaume (FR)
(74) Mandataire: CAPRI
(86) Numéro de dépôt international: PCT/FR2014/050619
(87) Numéro de publication internationale: WO 2014/147330

(56) Documents cités:
- WO-A1-2011/154658
- WO-A2-2009/136098
- FR-A1- 2 881 117
- FR-A1- 2 924 352
- US-A1- 2002 053 344

## Description

La présente invention concerne un dispositif de distribution de produit fluide, et plus particulièrement un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à disposer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est d'abord chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a chargé la dose (soit à partir d'un réservoir multidoses, soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Un autre problème qui peut se poser concerne l'assemblage de certaines pièces, notamment mobiles, qui doivent supporter des contraintes importantes en fonctionnement, et pour lesquels l'assemblage doit être particulièrement fiable pour éviter tout risque de dysfonctionnement. Avec la petite taille de certaines pièces, il peut être compliqué de garantir une telle fiabilité d'assemblage. Avec les inhalateurs qui sont chargés, par exemple lors de leur ouverture, puis déclenchés par l'inhalation, il est important d'éviter ou de limiter les risques de déclenchements intempestifs après chargement et avant inhalation, sans imposer un seuil d'inhalation trop élevé pour réaliser le déclenchement, qui pourrait être difficilement accessible à des personnes affaiblies. Un autre problème qui se pose avec les inhalateurs pourvus de bande de blisters est lié au déplacement de la bande, et au stockage de la partie utilisée de la bande. Ainsi, selon la longueur de la bande, un espace important peut s'avérer nécessaire et tout blocage de la bande de blisters peut empêcher le bon fonctionnement de l'inhalateur. Par ailleurs, lorsque le dispositif d'avancée de la bande tire en même temps sur l'extrémité avant de la bande pour éviter un mauvais enroulement, il peut se poser un problème au fur et à mesure des actionnements en raison notamment du diamètre de la bande usée enroulée qui augmente progressivement.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un tel dispositif qui soit simple et peu coûteux à fabriquer et à assembler, fiable à l'assemblage et en utilisation, garantissant une précision et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a donc pour objet un dispositif de distribution de produit fluide, comportant un corps, au moins un élément de capot monté pivotant sur ledit corps entre une position fermée et une position ouverte, ledit dispositif comportant une pluralité de réservoirs individuels contenant chacun une dose unique de produit fluide, tel que de la poudre, lesdits réservoirs individuels étant disposés sur une bande allongée, des moyens d'ouverture étant prévus pour ouvrir un réservoir individuel à chaque actionnement du dispositif, ledit dispositif comportant des moyens de support mobiles adaptés à déplacer un réservoir individuel contre lesdits moyens d'ouverture à chaque actionnement, lesdits moyens de support mobiles étant déplaçables entre une position de non-distribution et une position de distribution, le dispositif comportant un système d'enroulement de bande comportant un tambour et un organe d'actionnement, l'extrémité avant de ladite bande allongée étant fixée audit tambour et ledit organe d'actionnement étant rotatif par rapport au corps dans un premier sens de rotation lorsque lesdits moyens de support mobiles sont déplacés de leur position de non-distribution vers leur position de distribution et dans un second sens de rotation, inverse audit premier sens de rotation, lorsque lesdits moyens de support mobiles sont déplacés de leur position de distribution vers leur position de non-distribution, ledit organe d'actionnement faisant tourner ledit tambour uniquement lorsqu'il tourne dans ledit second sens de rotation.

Avantageusement, ledit tambour est bloqué en rotation dans ledit premier sens de rotation par des moyens anti-retour.

Avantageusement, lesdits moyens anti-retour comportent un ergot disposé sur une patte déformable, ledit ergot coopérant avec une première denture dudit tambour pour bloquer ledit tambour en rotation dans ledit premier sens de rotation, ladite patte déformable se déformant pour permettre une rotation dudit tambour dans ledit second sens de rotation.

Avantageusement, ledit organe d'actionnement comporte une patte flexible, ladite patte flexible coopérant avec une seconde denture dudit tambour pour entrainer ledit tambour en rotation dans ledit second sens de rotation, ladite patte flexible se déformant pour permettre une rotation dudit organe d'actionnement par rapport audit tambour dans ledit premier sens de rotation.

Avantageusement, ledit organe d'actionnement comporte un doigt rigide coopérant avec une ouverture de commande desdits moyens de support mobiles.

Avantageusement, le dispositif comporte un système de déclenchement par l'inhalation qui comporte une chambre d'air déformable coopérant avec un embout d'inhalation, et un élément déclencheur coopérant avec ladite chambre d'air, de sorte que lors d'une inhalation à travers ledit embout d'inhalation, ladite chambre d'air est déformée et ledit élément déclencheur actionne lesdits moyens d'ouverture, de sorte que lors d'une inhalation à travers l'embout d'inhalation, un réservoir est ouvert par lesdits moyens d'ouverture.

Avantageusement, lesdits moyens d'ouverture comportent un élément de perçage fixe par rapport audit corps principal, adapté à découper une paroi de fermeture du réservoir de telle sorte que la ou les partie(s) découpée(s) n'obstrue(nt) pas la ou les ouverture(s) formée(s).

Ces caractéristiques et avantages et d'autres de la présente invention apparaîtront plus clairement au cours de la description détaillée suivante, faite en référence aux dessins joints, donnés à titre d'exemples non limitatifs, sur lesquels
- la figure 1 est une vue schématique en section transversale d'un dispositif de distribution selon un mode de réalisation avantageux, en position fermée des capots, montrant certains éléments du dispositif de distribution,
- la figure 2 est une vue similaire à celle de la figure 1, montrant d'autres éléments du dispositif de distribution,
- la figure 3 est une vue similaire à celle de la figure 1, en position ouverte des capots, avant inhalation,
- la figure 4 est une vue similaire à celle de la figure 2, en position ouverte des capots, avant inhalation,
- la figure 5 est une vue similaire à celle de la figure 1, en position ouverte des capots, après inhalation,
- la figure 6 est une vue similaire à celle de la figure 2, en position ouverte des capots, après inhalation,
- la figure 7 est une vue similaire à celle de la figure 2, en position refermée des capots,
- la figure 8 est une vue schématique en perspective éclatée d'un système d'enroulement de bande selon une variante avantageuse,
- la figure 9 est une vue schématique en perspective de l'organe d'actionnement selon une variante avantageuse,
- la figure 10 est une vue schématique en perspective du tambour selon une variante avantageuse, vue de dessus,
- la figure 11 est une vue schématique en perspective du tambour selon une variante avantageuse, vue de dessous,
- la figure 12 est une vue schématique en perspective des moyens de support mobiles selon une variante avantageuse,
- la figure 13 est une vue schématique en perspective de la plaque de support selon une variante avantageuse, avec une vue de détail agrandie, et
- les figures 14 à 17 sont des vues schématiques en section transversale du système d'enroulement de bande, respectivement avant inhalation, pendant inhalation, après inhalation et pendant retour à la position de départ.

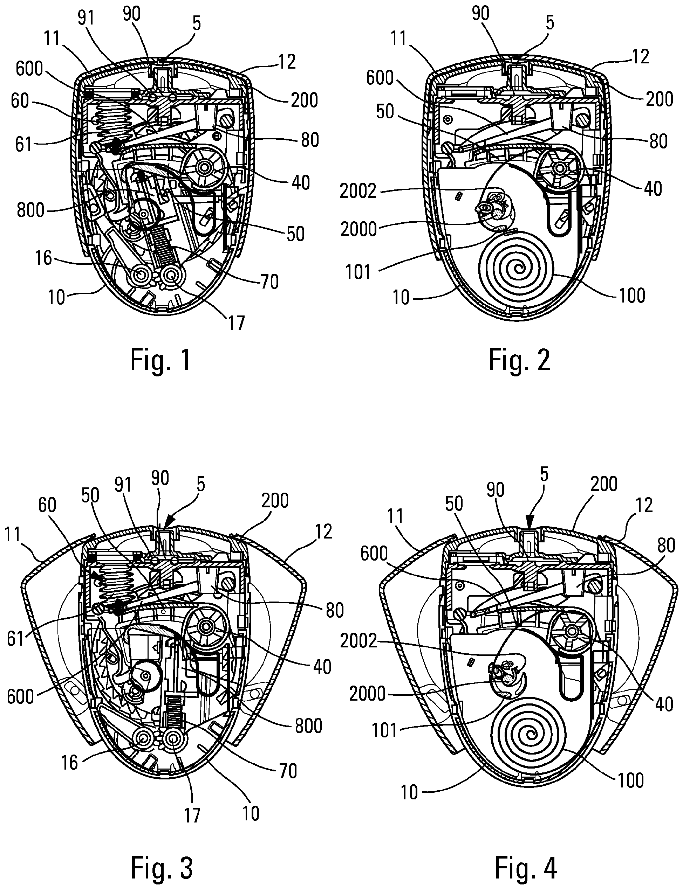

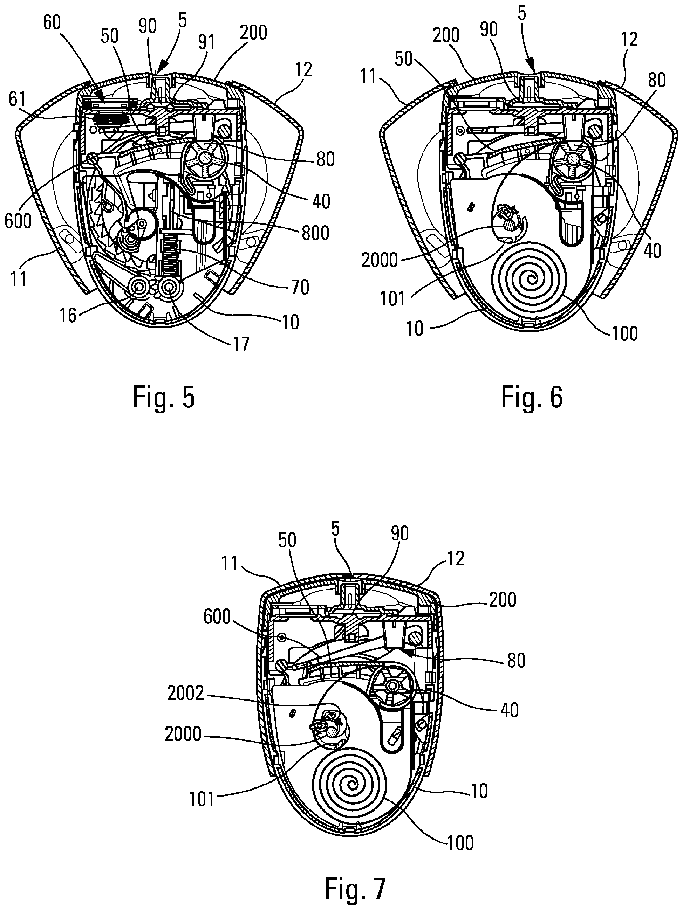

Sur les figures 1 à 7, il est représenté un exemple avantageux d'un inhalateur de poudre sèche. Dans des buts de clarté, tous les éléments du dispositif ne sont pas visibles sur toutes les figures. Cet inhalateur comporte un corps principal 10 sur lequel peuvent être montées coulissantes deux parties 11, 12 formant capot, adaptées à être ouvertes pour ouvrir et charger le dispositif. Le corps principal 10 peut être de forme environ arrondie comme représenté sur les figures, mais il pourrait avoir toute autre forme appropriée. Un embout buccal 200 est assemblé sur ledit corps 10. Cet embout buccal 200 définit un orifice de distribution 5 à travers lequel l'utilisateur va inhaler lors de l'actionnement du dispositif. Cet orifice de distribution 5 est typiquement disposé environ au centre de l'embout buccal 200. Les capots 11, 12 peuvent s'ouvrir par pivotement autour d'un axe de rotation commun, ou autour de deux axes parallèles en étant engrené l'un avec l'autre. Tout autre moyen pour ouvrir le dispositif est envisageable. En variante, le dispositif pourrait ne comporter qu'un seul capot au lieu de deux.

A l'intérieur du corps principal 10, il est prévu une bande allongée 100 de réservoirs individuels. La bande allongée 100 est visible sur les figures 2, 4, 6, 7 et 8. Les réservoirs individuels, également appelés blisters, ne sont pas représentés sur les figures dans des buts de clarté. Les blisters sont disposés les uns derrière les autres, de manière connue. Cette bande de blisters 100 est avantageusement constituée d'une couche ou paroi de base formant les cavités recevant les doses de poudre, et d'une couche ou paroi de fermeture qui obture de manière étanche chacun desdits blisters. Avant la première utilisation, la bande de blisters 100 peut être enroulée à l'intérieur du corps principal 10, de préférence dans une partie de stockage, et des moyens de déplacement de bande 40, notamment rotatifs, sont prévus pour progressivement dérouler et faire avancer cette bande de blisters 100.

La partie de bande comportant les blisters vides est avantageusement adaptée à s'enrouler dans un autre endroit dudit corps 10, de préférence une partie de réception, comme cela sera décrit plus en détail ci-après.

L'inhalateur comporte des moyens d'ouverture de blister 80 (seulement représentés très schématiquement dans des buts de clarté) comportant de préférence une aiguille de perçage et/ou de coupage de la couche de fermeture des blisters. De préférence, les moyens d'ouverture comportent un élément de perçage 80, fixe par rapport au corps principal 10, et contre lequel un blister respectif est déplacé à chaque actionnement par des moyens de support mobiles 50. Ces moyens de support mobiles 50, notamment pivotants sur le corps principal 10, sont prévus pour amener un blister respectif dans une position de distribution à chaque actionnement du dispositif. Ces moyens de support mobiles 50 sont pivotants entre une position de non-distribution et une position de distribution, dans laquelle un blister coopère avec lesdits moyens d'ouverture. Le blister est alors percé par ledit élément de perçage, qui pénètre dans ledit blister pour expulser la poudre au moyen du flux d'inhalation de l'utilisateur. Avantageusement, l'élément de perçage est adapté à découper une paroi de fermeture du réservoir de telle sorte que la ou les partie(s) découpée(s) n'obstrue(nt) pas la ou les ouverture(s) formée(s). Les documents WO 2006/079750 et WO 2009/007640 décrivent de tels moyens d'ouverture de blister, et sont donc intégrés dans la présente description à titre de références.

Les moyens de déplacement 40 sont adaptés à faire avancer la bande de blisters après chaque inhalation de l'utilisateur. Les moyens de support mobiles 50 sont adaptés à déplacer le blister à vider contre lesdits moyens d'ouverture 80 lors de l'actionnement, lors de chaque inhalation. Ces moyens de support mobiles peuvent être sollicités par un élément élastique 70, tel qu'un ressort ou tout autre élément élastique équivalent, ledit élément élastique pouvant être pré-chargé lors de l'ouverture du dispositif.

De préférence, les moyens de déplacement 40 sont formés par une roue d'indexage qui reçoit et guide la bande de blisters. La suite de la description sera donc faite en référence à une telle roue d'indexage 40. Une rotation de cette roue d'indexage 40 fait avancer la bande de blisters. Avant chaque inhalation, un blister plein est toujours en position face aux moyens d'ouverture 80. Les moyens de support mobiles 50 peuvent comporter un organe pivotant autour d'un axe de rotation, ladite roue d'indexage 40 étant avantageusement montée rotative sur ledit organe pivotant.

Un cycle d'actionnement du dispositif peut être le suivant. Lors de l'ouverture du dispositif, les deux parties latérales 11, 12 formant capot sont écartées l'une de l'autre en pivotant sur le corps pour ouvrir le dispositif, et ainsi charger le dispositif. Dans cette position la roue d'indexage 40 ne peut pas se déplacer vers l'élément de perçage 80 car les moyens de support mobiles 50 sont retenus par des moyens de blocage appropriés (non représentés dans des buts de clarté). Les documents WO 2009/077700 et WO 2009/136098 décrivent de tels moyens de blocage, et sont donc intégrés dans la présente description à titre de références. Lors de l'inhalation par l'utilisateur à travers l'embout buccal, ces moyens de blocage sont débloqués, ce qui provoque alors le pivotement des moyens de support mobiles 50 et donc le déplacement de ladite roue d'indexage 40 en direction de l'aiguille, et donc l'ouverture d'un blister.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture, on prévoit un système de déclenchement par l'inhalation 60 qui comporte avantageusement une chambre d'air 61 déformable sous l'effet de l'inhalation, cette chambre d'air étant adaptée à libérer les moyens de blocage. La chambre d'air 61 peut avantageusement être réalisée en forme de soufflet. L'inhalation de l'utilisateur provoque la déformation de ladite chambre d'air déformable, libérant ainsi lesdits moyens de blocage et permettant donc le déplacement des moyens de support mobiles, et donc d'un blister respectif, vers sa position d'ouverture. Le blister n'est donc ouvert qu'au moment de l'inhalation, de sorte qu'il est simultanément vidé. Il n'y a donc aucun risque de perte de dose entre l'ouverture du blister et son vidage.

L'inhalateur des figures 1 à 7 comporte en outre une chambre de dispersion 90 qui est destinée à recevoir la dose de poudre après ouverture d'un blister respectif. Avantageusement, cette chambre de dispersion est pourvue d'au moins une bille 91, de préférence plusieurs, qui se déplace(nt) à l'intérieur de ladite chambre 90 pendant l'inhalation, notamment pour améliorer la distribution du mélange air et poudre après ouverture d'un blister, afin d'augmenter l'efficacité du dispositif.

Après l'inhalation, lorsque l'utilisateur referme le dispositif, tous les composants reviennent vers leurs positions de repos initiales. Le dispositif est alors prêt pour un nouveau cycle d'utilisation.

Selon un aspect avantageux de l'inhalateur, les blisters sont formés sur une bande allongée souple, qui, au début, est principalement stockée sous forme de bobine dans un logement de stockage à l'intérieur du corps principal 10 du dispositif. Avantageusement, cette bande de blisters enroulée est maintenue par des parois internes dudit logement de stockage sans que son extrémité arrière (dans le sens d'avancement de la bande de blisters) ne soit fixée par rapport audit corps principal 10, ce qui permet un assemblage plus aisé de cette bobine de bande de blisters à l'intérieur du dispositif. La bande de blisters est déplacée au moyen de la roue d'indexage 40 qui présente avantageusement au moins un, de préférence plusieurs évidements, dont la forme correspond à celle des blisters. Ainsi, lorsque cette roue d'indexage 40 tourne, elle fait avancer la bande de blisters. Bien entendu en variante ou de manière additionnelle, on pourrait utiliser d'autres moyens d'avancée de bande de blisters, par exemple en prévoyant un profil sur les bords longitudinaux latéraux de la bande de blisters, ledit profil étant adapté à coopérer avec des moyens d'entraînement appropriés. De même, des trous ménagés le long des bords latéraux de la bande de blisters pourraient également être utilisés pour faire avancer la bande de blisters au moyen de roues dentées coopérant avec lesdits trous.

Après ouverture d'un ou plusieurs blister(s), la partie de bande de blisters avec les blisters vidés, c'est-à-dire la partie avant de ladite bande 100, doit pouvoir être stockée de manière aisée et non encombrante dans le dispositif, en évitant tout risque de blocage. Avantageusement, cette bande de blisters usée s'enroule automatiquement sur elle-même pour à nouveau former une bobine.

Selon encore un autre aspect de l'inhalateur, un dispositif de comptage ou d'indication de doses (non représenté dans des buts de clarté) peut également être prévu. Ce dispositif peut comporter des chiffres ou symboles inscrits directement sur la bande de blister et visibles à travers une fenêtre appropriée dans le corps principal 10 du dispositif. En variante, on pourrait envisager d'utiliser un compteur avec un ou plusieurs disque(s) ou anneau(x) rotatif(s) comportant des numéros ou symboles. Les documents WO 2008/012458 et WO 2011/154659 décrivent de tels compteurs. Un but de l'invention est d'éviter de compter des doses qui ne sont pas distribuées, par exemple en cas d'erreur de manipulation, ou d'une manipulation incomplète du dispositif. Il est donc souhaitable que le compteur ou indicateur ne soir actionné qu'une fois que l'utilisateur a inhalé, puisque c'est cette inhalation qui conditionne l'ouverture et la distribution de la dose de chaque blister. Avantageusement, l'actionnement du compteur se fait donc après inhalation, lorsque l'utilisateur referme le dispositif.

Un des éléments de capot, par exemple l'élément de capot mobile 12, est solidaire d'un organe d'armement 800 qui peut coulisser dans un logement approprié. Cet organe d'armement 800 est avantageusement pivotant par rapport audit corps 10 ensemble avec l'élément de capot 12. Cet organe d'armement 800 peut se déplacer contre le ressort 70, avantageusement un ressort à boudins. L'organe d'armement 800 est donc connecté d'un côté audit ressort 70 et de l'autre côté, il coopère avec les moyens de support mobiles 50, en particulier avec un organe pivotant sur le corps 10, et sur lequel est fixée de manière rotative la roue d'indexage 40.

Lorsque l'élément de capot mobile 12 est ouvert, l'organe d'armement 800 est déplacé en comprimant le ressort 70. L'organe pivotant des moyens de support mobiles 50 est lui empêché de se déplacer par les moyens de blocage susmentionnés, qui ne seront libérés qu'au moment de l'inhalation. Ainsi, en l'absence d'inhalation dans la position ouverte, la fermeture des éléments de capot 11, 12 provoquerait simplement le retour à la position de repos pour l'organe d'armement 800, et la décompression du ressort 70.

Ainsi, lorsque l'utilisateur ouvre l'inhalateur il charge le système. S'il n'inhale pas et qu'il referme l'inhalateur, celui-ci reprend simplement sa position de départ sans déplacer la bande de blisters ni les moyens de blocage. Il n'y a donc aucun risque qu'un blister (et donc une dose de produit actif) soit perdu par un actionnement malencontreux ou incomplet dans lequel l'utilisateur n'exercerait pas d'inhalation entre l'ouverture et la fermeture. L'ouverture du blister, son vidage, la distribution de la poudre dans les poumons de l'utilisateur, le déplacement de la bande de blisters pour amener un nouveau blister plein en face des moyens d'ouverture ainsi que le comptage de la dose ne sont donc possibles que si l'utilisateur inhale.

Les moyens de blocage, qui avant inhalation bloquent les moyens de support mobiles 50 et notamment l'organe pivotant qui coopère avec l'organe d'armement, sont connectés à la chambre d'air déformable 61 sensible à l'inhalation de l'utilisateur par l'intermédiaire d'un élément déclencheur 600, de sorte que lors de l'inhalation de l'utilisateur, ladite chambre d'air déformable se déforme, faisant pivoter l'élément déclencheur 600, et libérant ainsi lesdits moyens de blocage. Ceci permet le déplacement desdits moyens de support mobiles 50 vers leur position de distribution sous l'effet de la force exercée par le ressort comprimé 70 sur l'organe d'armement 800 qui pousse sur l'organe pivotant 50. Ce déplacement provoque l'ouverture d'un blister plein et la distribution d'une dose.

Une surface de came est formée sur lesdits moyens de support mobiles 50, sur laquelle glisse l'organe d'armement 800. L'organe d'armement 800 est donc adaptée à comprimer le ressort 70 lorsque l'élément de capot 12 est ouvert, et à décomprimer ledit ressort 70 lorsque ledit élément de capot 12 est refermé. Avantageusement, l'organe d'armement 800 comporte, dans sa partie en contact avec la surface de came, une partie arrondie pour favoriser le glissement de l'organe d'armement 800 sur ladite surface de came.

Après l'inhalation, c'est-à-dire en position de distribution, les moyens de blocage ont été libérés et les moyens de support mobiles 50 ont été déplacés vers le haut par le ressort comprimé 70.

Avantageusement, les deux éléments de capot mobiles 11, 12 sont engrenés l'un dans l'autre via des engrenages appropriés pour garantir une ouverture et une fermeture symétrique desdits deux éléments de capot mobiles. Cet engrenage peut se faire à proximité de leur axe de pivotement 16, 17.

Selon l'invention, il est prévu un système d'enroulement de bande pour assurer l'enroulement correct de la partie de bande comportant les blisters vides, c'est-à-dire la partie avant dans le sens d'avancement de la bande de blisters dans le dispositif.

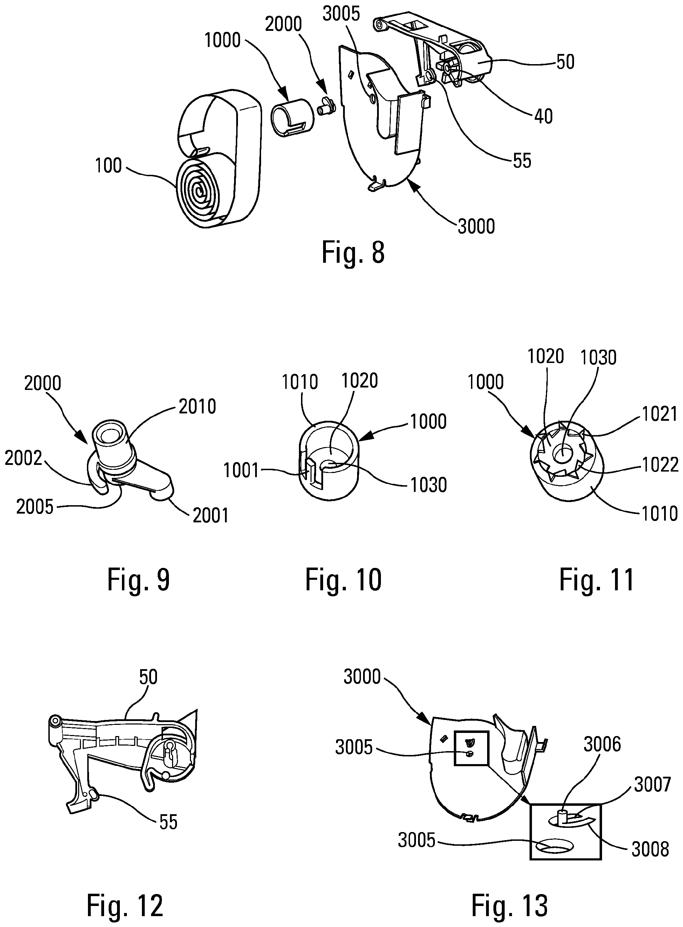

Les figures 8 à 13 illustrent les différents composants selon une variante avantageuse dudit système d'enroulement. Dans cette variante, l'extrémité avant 101 de la bande de blisters 100, dans le sens d'avancement de ladite bande 100, est fixée à un tambour rotatif 1000, notamment sur un doigt 1001 dudit tambour 1000. Ledit tambour comporte un manchon creux 1010 dont un coté axial est fermé par une paroi axiale 1020 pourvue d'une ouverture centrale 1030. Le doigt 1001 est avantageusement formé dans ledit manchon 1010. La paroi axiale 1020 comporte sur sa face externe une première denture 1021 et une seconde denture 1022, lesdites dentures faisant saille axialement hors de ladite paroi axiale 1020.

Un organe d'actionnement 2000 est disposé de manière concentrique ou coaxiale contre ladite face externe de ladite paroi axiale 1020 du tambour 1000. Cet organe d'actionnement 2000 comporte un premier plot axial 2010 qui traverse ladite ouverture centrale 1030 du tambour pour s'étendre axialement à l'intérieur dudit manchon 1010 dudit tambour 1000. Du coté axial opposé, ledit organe d'actionnement 2000 comporte un second plot axial 2005, qui va coopérer avec une ouverture 3005 formée dans une plaque de support 3000 solidaire du corps 10. Ledit second plot axial 2005 dudit organe d'actionnement 2000 est monté rotatif dans ladite ouverture 3005. Il comporte un doigt rigide 2001 adapté à coopérer avec une ouverture de commande 55 desdits moyens de support mobiles 50. L'organe d'actionnement 2000 comporte aussi une patte flexible 2002 adaptée à coopérer avec ladite seconde denture 1022 dudit tambour 1000. Ledit organe d'actionnement 2000 est rotatif par rapport au corps 10 dans un premier sens de rotation lorsque lesdits moyens de support mobiles 50 sont déplacés de leur position de non-distribution vers leur position de distribution et dans un second sens de rotation, inverse audit premier sens de rotation, lorsque lesdits moyens de support mobiles 50 sont déplacés de leur position de distribution vers leur position de non-distribution.

La plaque de support 3000 comporte un ergot saillant 3006 formé sur une patte déformable 3007 définie par une découpe en U 3008 dans ladite plaque de support 3000. Cet ergot 3006 est adapté à coopérer avec ladite première denture 1021 du tambour 1000.

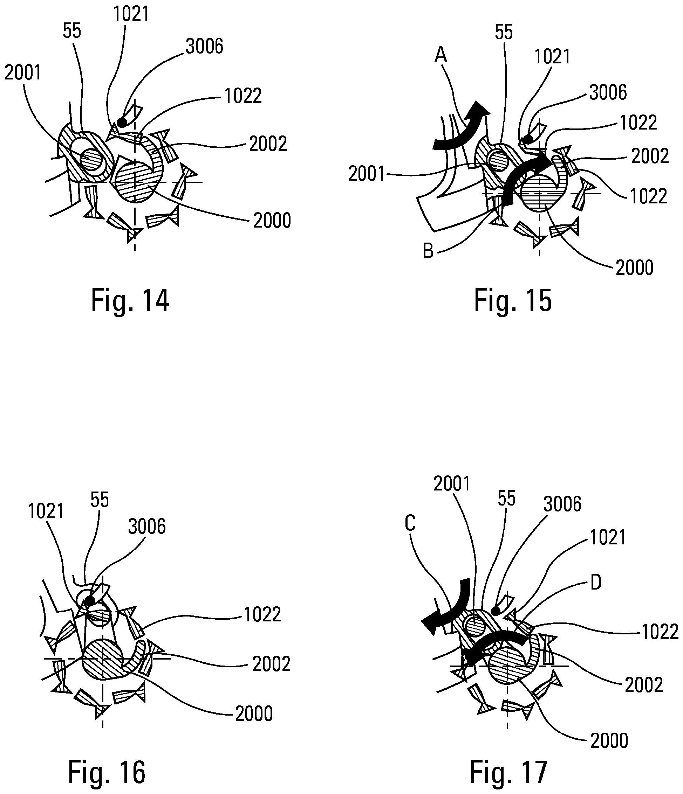

Le fonctionnement du système d'enroulement va être décrit en référence aux figures 14 à 17.

La figure 14 montre la position de repos, avant actionnement.

Lorsque l'utilisateur inhale, les moyens de support mobiles 50 se déplacent par rapport au corps 10 et donc par rapport à la plaque support 3000, dans le sens de la flèche A sur la figure 15. L'ouverture de commande 55 desdits moyens de support mobiles 50 entraine ainsi le doigt rigide 2001 de l'organe d'actionnement 2000, ce qui provoque la rotation de l'organe d'actionnement 2000 dans le sens de la flèche B sur la figure 15, qui est le premier sens de rotation. La patte flexible 2002 de l'organe d'actionnement 2000 glisse sur une dent de la seconde denture 1022, en se déformant, jusqu'à venir s'encliqueter derrière la prochaine dent de ladite seconde denture 1022, comme visible sur la figure 16. Pendant ce temps, l'ergot 3006 de la plaque de support 3000 bloque toute rotation du tambour dans le sens de la flèche B, en coopérant avec une dent de la première denture 1021 du tambour 1000. Ainsi, pendant l'inhalation, l'organe d'actionnement 2000 tourne dans ledit premier sens de rotation, mais ledit tambour 1000 reste immobile, bloqué par l'ergot 3006 formant moyens anti-retour.

Lorsque les moyens de support mobiles 50 sont ramenés vers leur position initiale, après inhalation, l'ouverture de commande 55 pivote donc en sens inverse dans le sens de la flèche C sur la figure 17, et entraine le doigt rigide 2001 de l'organe d'actionnement 2000. Ceci provoque une rotation de l'organe d'actionnement 2000 dans le sens de la flèche D, qui est le second sens de rotation, inverse au premier sens de rotation. Lors de cette rotation, la patte flexible 2002 pousse sur une dent de la seconde denture 1022 du tambour, faisant ainsi tourner ledit tambour 1000 également dans le sens de la flèche D. Ceci enroule la bande de blister 100 autour dudit tambour 1000. Pendant cette rotation du tambour 1000, la patte déformable 3007 se déforme et l'ergot 3006 de la plaque de support 3000 peut glisser sur une dent de la première denture 1021, pour venir s'encliqueter derrière la prochaine dent de ladite première denture 1021.

Le dispositif est alors prêt pour un autre cycle d'actionnement.

La présente invention permet donc d'assurer un enroulement fiable de la partie usée de la bande de blister, cet enroulement étant assuré lors du retour des moyens de support mobiles 50 vers leur position de non-distribution, après l'inhalation. Ceci garantit un enroulement fiable, et donc un fonctionnement fiable du dispositif d'inhalation.

La présente invention permet donc de fournir un inhalateur de poudre sèche qui procure notamment une ou plusieurs des fonctions suivantes :
▪ une pluralité de doses individuelles de poudre stockées dans des blisters individuels étanches, par exemple 30 ou 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération pré-chargé ;
▪ des moyens d'entraînement de forme appropriée en prises avec les blisters pour réaliser le déplacement de la bande de blisters après chaque inhalation, et amener un nouveau blister plein dans une position dans laquelle il est destiné à être ouvert par les moyens d'ouverture appropriés ;
▪ des moyens pour éviter une perte de dose en cas d'ouverture de l'inhalateur mais en l'absence d'inhalation ;
▪ des moyens pour assurer un enroulement fiable de la partie de bande de blister comportant les blisters vides ;
▪ un indicateur de doses adapté à compter les doses seulement en cas d'inhalation.

D'autres fonctions sont également fournies par le dispositif de l'invention tel que cela a été décrit précédemment.

Il est à noter que les différentes fonctions, même si elles ont été représentées comme prévues simultanément sur l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les blisters individuels sont arrangés les uns par rapport aux autres, etc. Les moyens d'armement et le système de déclenchement par l'inhalation pourraient être réalisés différemment. Il en est de même des autres parties constitutives du dispositif.

Diverses modifications sont également possibles pour un homme du métier sans sortir du cadre de la présente invention tel que défini par les revendications annexées. En particulier, les diverses caractéristiques et fonctionnalités du dispositif décrites en référence aux dessins peuvent être combinées entre elles d'une quelconque façon appropriée.

## Revendications

1. Dispositif de distribution de produit fluide, comportant un corps (10), au moins un élément de capot (11, 12) monté pivotant sur ledit corps (10) entre une position fermée et une position ouverte, ledit dispositif comportant une pluralité de réservoirs individuels contenant chacun une dose unique de produit fluide, tel que de la poudre, lesdits réservoirs individuels étant disposés sur une bande allongée (100), des moyens d'ouverture (80) étant prévus pour ouvrir un réservoir individuel à chaque actionnement du dispositif, ledit dispositif comportant des moyens de support mobiles (50) adaptés à déplacer un réservoir individuel contre lesdits moyens d'ouverture (80) à chaque actionnement, lesdits moyens de support mobiles (50) étant déplaçables entre une position de non-distribution et une position de distribution, **caractérisé en ce que** le dispositif comporte un système d'enroulement de bande comportant un tambour (1000) et un organe d'actionnement (2000), l'extrémité avant (101) de ladite bande allongée (100) étant fixée audit tambour (1000) et ledit organe d'actionnement (2000) étant rotatif par rapport au corps (10) dans un premier sens de rotation lorsque lesdits moyens de support mobiles (50) sont déplacés de leur position de non-distribution vers leur position de distribution et dans un second sens de rotation, inverse audit premier sens de rotation, lorsque lesdits moyens de support mobiles (50) sont déplacés de leur position de distribution vers leur position de non-distribution, ledit organe d'actionnement (2000) faisant tourner ledit tambour (1000) uniquement lorsqu'il tourne dans ledit second sens de rotation.

2. Dispositif selon la revendication 1, dans lequel ledit tambour (1000) est bloqué en rotation dans ledit premier sens de rotation par des moyens anti-retour (3006, 3007).

3. Dispositif selon la revendication 2, dans lequel lesdits moyens anti-retour comportent un ergot (3006) disposé sur une patte déformable (3007), ledit ergot (3006) coopérant avec une première denture (1021) dudit tambour (1000) pour bloquer ledit tambour en rotation dans ledit premier sens de rotation, ladite patte déformable (3007) se déformant pour permettre une rotation dudit tambour dans ledit second sens de rotation.

4. Dispositif selon l'une quelconque des revendications précédentes, dans lequel ledit organe d'actionnement (2000) comporte une patte flexible (2002), ladite patte flexible (2002) coopérant avec une seconde denture (1022) dudit tambour (1000) pour entrainer ledit tambour en rotation dans ledit second sens de rotation, ladite patte flexible (2002) se déformant pour permettre une rotation dudit organe d'actionnement (2000) par rapport audit tambour (1000) dans ledit premier sens de rotation.

5. Dispositif selon l'une quelconque des revendications précédentes, dans lequel ledit organe d'actionnement (2000) comporte un doigt rigide (2001) coopérant avec une ouverture de commande (55) desdits moyens de support mobiles (50).

6. Dispositif selon l'une quelconque des revendications précédentes, comportant un système de déclenchement par l'inhalation (60) qui comporte une chambre d'air déformable (61) coopérant avec un embout d'inhalation (200), et un élément déclencheur (600) coopérant avec ladite chambre d'air (61), de sorte que lors d'une inhalation à travers ledit embout d'inhalation (200), ladite chambre d'air (61) est déformée et ledit élément déclencheur (600) actionne lesdits moyens d'ouverture (80), de sorte que lors d'une inhalation à travers l'embout d'inhalation (200), un réservoir est ouvert par lesdits moyens d'ouverture.

7. Dispositif selon l'une quelconque des revendications précédentes, dans lequel lesdits moyens d'ouverture (80) comportent un élément de perçage fixe par rapport audit corps principal (10), adapté à découper une paroi de fermeture du réservoir de telle sorte que la ou les partie(s) découpée(s) n'obstrue(nt) pas la ou les ouverture(s) formée(s).

## Patentansprüche

1. Ausgabevorrichtung für ein fluides Produkt, aufweisend einen Körper (10), mindestens ein Abdeckelement (11, 12), das auf dem Körper (10) zwischen einer geschlossenen Position und einer geöffneten Position schwenkbar montiert ist, wobei die Vorrichtung mehrere einzelne Behälter aufweist, die jeweils eine einzelne Dosis von fluidem Produkt, wie Pulver, enthalten, wobei die einzelnen Behälter auf einem langgestreckten Streifen (100) angeordnet sind, wobei Öffnungsmittel (80) vorgesehen sind, um bei jeder Betätigung der Vorrichtung einen einzelnen Behälter zu öffnen, wobei die Vorrichtung bewegliche Trägermittel (50) aufweist, die dazu geeignet sind, bei jeder Betätigung einen einzelnen Behälter gegen die Öffnungsmittel (80) zu verschieben, wobei die beweglichen Trägermittel (50) zwischen einer Nichtausgabe-Position und einer Ausgabeposition verschiebbar sind, **dadurch gekennzeichnet, dass** die Vorrichtung ein Streifenabrollsystem aufweist, welches eine Trommel (1000) und eine Betätigungseinrichtung (2000) aufweist, wobei das vordere Ende (101) des langgestreckten Streifens (100) an der Trommel (1000) befestigt ist und die Betätigungseinrichtung (2000) relativ zum Körper (10) in eine erste Drehrichtung drehbar ist, wenn die beweglichen Trägermittel (50) von ihrer Nichtausgabe-Position in ihre Ausgabeposition verschoben werden, und in eine zweite Drehrichtung drehbar ist, die zur ersten Drehrichtung entgegengesetzt ist, wenn die beweglichen Trägermittel (50) von ihrer Ausgabeposition in ihre Nichtausgabe-Position verschoben werden, wobei die Betätigungseinrichtung (2000) die Trommel (1000) nur dann in Drehung versetzt, wenn sie sich in die zweite Drehrichtung dreht.

2. Vorrichtung nach Anspruch 1, wobei die Trommel (1000) in der ersten Drehrichtung durch Rückdrehsicherungsmittel (3006, 3007) gegen Drehung gesperrt ist.

3. Vorrichtung nach Anspruch 2, wobei die Rückdrehsicherungsmittel einen Vorsprung (3006) aufweisen, der auf einer verformbaren Lasche (3007) angeordnet ist, wobei der Vorsprung (3006) mit einer ersten Verzahnung (1021) der Trommel (1000) zusammenwirkt, um die Trommel in der ersten Drehrichtung gegen eine Drehung zu sperren, wobei sich die verformbare Lasche (3007) verformt, um eine Drehung der Trommel in die zweite Drehrichtung zu gestatten.

4. Vorrichtung nach einem der vorhergehenden Ansprüche, wobei die Betätigungseinrichtung (2000) eine flexible Lasche (2002) aufweist, wobei die flexible Lasche (2002) mit einer zweiten Verzahnung (1022) der Trommel (1000) zusammenwirkt, um die Trommel drehend in die zweite Drehrichtung mitzunehmen, wobei sich die flexible Lasche (2002) verformt, um eine Drehung der Betätigungsreinrichtung (2000) relativ zur Trommel (1000) in die erste Drehrichtung zu gestatten.

5. Vorrichtung nach einem der vorhergehenden Ansprüche, wobei die Betätigungsreinrichtung (2000) einen starren Zapfen (2001) aufweist, der mit einer Steueröffnung (55) der beweglichen Trägermittel (50) zusammenwirkt.

6. Vorrichtung nach einem der vorhergehenden Ansprüche, aufweisend ein Auslösesystem durch Inhalation (60), welches eine verformbare Luftkammer (61) aufweist, die mit einem Inhalationsansatz (200) zusammenwirkt, und ein Auslöseelement (600), das mit der Luftkammer (61) derart zusammenwirkt, dass bei einer Inhalation durch den Inhalationsansatz (200) die Luftkammer (61) verformt wird und das Auslöseelement (600) die Öffnungsmittel (80) derart betätigt, dass bei einer Inhalation durch den Inhalationsansatz (200) ein Behälter durch die Öffnungsmittel geöffnet wird.

7. Vorrichtung nach einem der vorhergehenden Ansprüche, wobei die Öffnungsmittel (80) ein Durchdringungselement aufweisen, welches relativ zum Hauptkörper (10) fixiert und dafür geeignet ist, eine Verschlusswand des Behälters derart abzuschneiden, dass der abgeschnittene Teil oder die abgeschnittenen Teile nicht die gebildete(n) Öffnung(en) blockiert (blockieren).

## Claims

1. A fluid dispenser device including a body (10) and at least one cover element (11, 12) that is mounted to pivot on said body (10) between a closed position and an open position, said device including a plurality of individual reservoirs each containing a single dose of fluid, such as powder, said individual reservoirs being arranged on an elongate strip (100), opening means (80) being provided for opening an individual reservoir each time the device is actuated, said device including movable support means (50) that are adapted to move an individual reservoir against said opening means (80) on each actuation, said movable support means (50) being movable between a non-dispensing position and a dispensing position, the device being **characterized in that** it includes a strip-rolling system comprising a drum (1000) and an actuator member (2000), the leading end (101) of said elongate strip (100) being fastened to said drum (1000), and said actuator member (2000) turning relative to the body (10) in a first turning direction when said movable support means (50) are moved from their non-dispensing position towards their dispensing position, and in a second turning direction, opposite to said first turning direction, when said movable support means (50) are moved from their dispensing position towards their non-dispensing position, said actuator member (2000) causing said drum (1000) to turn only when turning in said second turning direction.

2. A device according to claim 1, wherein said drum (1000) is prevented from turning in said first turning direction by non-return means (3006, 3007).

3. A device according to claim 2, wherein said non-return means comprise a lug (3006) that is arranged on a deformable tab (3007), said lug (3006) co-operating with a first set of teeth (1021) of said drum (1000) so as to prevent said drum from turning in said first turning direction, said deformable tab (3007) deforming so as to enable said drum to turn in said second turning direction.

4. A device according to any preceding claim, wherein said actuator member (2000) includes a flexible tab (2002), said flexible tab (2002) co-operating with a second set of teeth (1022) of said drum (1000) so as to turn said drum in said second turning direction, said flexible tab (2002) deforming so as to enable said actuator member (2000) to turn relative to said drum (1000) in said first turning direction.

5. A device according to any preceding claim, wherein said actuator member (2000) includes a rigid finger (2001) that co-operates with a control opening (55) in said movable support means (50).

6. A device according to any preceding claim, including an inhalation trigger system (60) that comprises a deformable air chamber (61) that co-operates with an inhalation piece (200), and a trigger element (600) that co-operates with said air chamber (61), such that during inhalation through said inhalation piece (200), said air chamber (61) is deformed and said trigger element (600) actuates said opening means (80), such that during inhalation through the inhalation piece (200), a reservoir is opened by said opening means.

7. A device according to any preceding claim, wherein said opening means (80) include a perforator element that is stationary relative to said main body (10) and that is adapted to cut a closure wall of the reservoir in such a manner that the cut portion(s) does/do not obstruct the opening(s) that is/are formed.
